(19) **Europäisches Patentamt / European Patent Office / Office européen des brevets**

(11) **EP 4 215 170 A1**

(12) **EUROPEAN PATENT APPLICATION**

(43) Date of publication:
**26.07.2023 Bulletin 2023/30**

(21) Application number: **22152553.8**

(22) Date of filing: **20.01.2022**

(51) International Patent Classification (IPC):
**A61F 13/20** $^{(2006.01)}$    **D04H 1/4258** $^{(2012.01)}$
**D04H 3/013** $^{(2012.01)}$

(52) Cooperative Patent Classification (CPC):
**D04H 3/013; A61F 13/2071; D04H 1/4258**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **LENZING AKTIENGESELLSCHAFT**
**4860 Lenzing (AT)**

(72) Inventors:
- **Gregorich, Katharina**
  **4813 Altmünster (AT)**
- **Goldhalm, Gisela**
  **3363 Neufurth (AT)**

(74) Representative: **Global Patent Management**
**Lenzing AG**
**Werkstraße 2**
**4860 Lenzing (AT)**

(54) **NONWOVEN LAYER COMPRISING A NETWORK OF SUBSTANTIALLY CONTINUOUS REGENERATED CELLULOSIC FIBERS**

(57) Nonwoven layer comprising a network of substantially continuous regenerated cellulosic fibers. The nonwoven layer has a basis weight from about 10 to about 30 g/m$^2$, preferably from about 15 to about 25 g/m$^2$ and an average maximal pore length between 50 $\mu$m and 500 $\mu$m, preferably between 100 and 400 $\mu$m.

Fig. 1

EP 4 215 170 A1

**Description**

**Field of the invention**

The present disclosure relates to the field of cellulosic nonwoven materials and especially to nonwoven materials having good retention properties, specifically lint retention properties. Such nonwoven materials can, for example, be used as a tampon overwrap.

**Description of the Related Art**

The current disclosure relates to improvements concerning the production and use of nonwoven layers comprising a network (also called "web") of substantially continuous regenerated cellulosic fibers. More specifically, the current disclosure relates to novel nonwoven layers that are produced according to the so-called solution-blown process and have improved lint retention properties.

Cellulosic fibres can be produced by various processes. In one embodiment a lyocell fibre is spun from a lyocell spinning solution comprising cellulose dissolved in N-methyl morpholine N-oxide (NMMO) by a meltblown process, in principle known from e.g. EP 1093536 B1, EP 2013390 B1 and EP 2212456 B1. In jurisdictions where this is possible, the documents EP 1093536 B1, EP 2013390 B1 and EP 2212456 B1 are included herein by reference.

Where the term meltblown is used, it will be understood that it refers to a process that is similar or analogous to the process used for the production of synthetic thermoplastic fibres (filaments are extruded under pressure through nozzles and stretched to required degree by high velocity/high temperature extension air flowing substantially parallel to the filament direction), even though the cellulose is dissolved in solution (i.e. not a molten thermoplastic) and the spinning & air temperatures are only moderately elevated. Therefore the term "solution blown" may be even more appropriate here instead of the term "meltblown" which has already become somewhat common for these kinds of technologies. For the purposes of the present disclosure the terms "meltblown" and "solution blown" are used synonymously, wherein the term "meltblown" is rather used as a generic term, independent of the fiber material and the term "solution blown" is more specifically used for the production of cellulosic nonwovens from lyocell spinning solution.

It is also known to use other solvents than NMMO, such as ionic liquids or the like, to prepare the spinning solution. Spinning solutions that are prepared using such alternative solvents also fall under the scope of the term "lyocell spinning solution", as it is used herein.

In one embodiment for making a nonwoven web the fibers are contacted with a nonsolvent such as water (or water/NMMO mixture) by spraying, after extrusion but before web formation. The fibers are subsequently taken up on a moving foraminous support to form a nonwoven web, washed and dried.

Freshly-extruded lyocell solution ('solvent spun', which will contain only, for example, 5-15% cellulose) behaves in a similar way to 'sticky' and deformable thermoplastic filaments. Causing the freshly-spun filaments to contact each other while still swollen with solvent and with a 'sticky' surface under even low pressure will cause merged filament bonding, where molecules from one filament mix irreversibly with molecules from a different filament. Once the solvent is removed and coagulation of filaments completed, this type of bonding is impossible.

Processes for the production of a nonwoven layer comprising a network of substantially continuous regenerated cellulosic fibers are documented and published in detail in WO 2007/124521 A1, WO 2018/071928 A1, WO 2018/184941 A1 and WO 2020/016296 A1 and the literature cited therein. In jurisdictions where this is possible, the documents WO 2007/124521 A1, WO 2018/071928 A1, WO 2018/184941 A1 and WO 2020/016296 A1 are included herein by reference.

Typically, tampons with absorbent cores made of Viscose, Cotton or Organic Cotton that are found on the market can (but not necessarily must) contain an overwrap, which can be a nonwoven cover or an overwrap fabric.

Without an overwrap, tampons can show high amounts of fiber lint in wet state, also referred to as shedding. The term "fiber lint", as it is used herein, describes the release of fibrous material. Obviously, fiber lint is a highly undesirable attribute for tampons.

Some cotton fiber based tampons have no need for an overwrap, as the cotton fibers used in the absorbent core show only low fiber lint. Nonetheless, these tampons can only meet the demands of rather marginal areas of the whole market.

Typically, nonwoven materials that are used as a tampon overwrap are produced with synthetic fibers, such as polyethylene, polyester or polypropylene, and have a rather low basis weight, generally in the range of about 15 $g/m^2$ to about 25 $g/m^2$.

To date, cellulosic nonwoven materials were only used as a top-sheet for hygiene articles other than tampons. For example, EP3730111A1 discloses a top-sheet for use in an absorbent hygiene article comprising at least one nonwoven layer, and an adsorbent hygiene article containing said top-sheet. The term "top-sheet", as it is used in this context and as it is also used herein, simply denotes the outermost layer of the hygiene article. Such cellulosic top-sheets serve as a leakage protection and distribution layer. Nonetheless, cellulosic nonwoven top-sheets, for example made with carding/hydroentangling, show a high fabric inhomogeneity, especially in the low basis weight range. This leads to larger holes in the nonwoven fabric so that the fabric provides no protection from fiber lint.

Wetlaid fabrics, on the other hand, are too stiff and paperlike to be used as a top-sheet or overwrap.

The term "overwrap", as it is used herein, denotes a material layer that is arranged on the outside of an absorbent core, especially on the outside of a tampon absorbent core, to prevent fiber lint. The overwrap can be, but not necessarily must be, the outermost layer of the tampon. For example, the overwrap can still be covered by a top-sheet. In some cases, the overwrap can also serve as a top-sheet, i.e. be the outermost layer of the tampon.

## Summary

The present disclosure provides novel cellulosic nonwoven layers providing for an advantageous fiber lint protection. The nonwoven materials disclosed herein can be used, inter alia, as a tampon overwrap.

According to one embodiment the present disclosure relates to a nonwoven layer comprising a network of substantially continuous regenerated cellulosic fibers, wherein the nonwoven layer has a basis weight from about 10 to about 30 g/m², preferably from about 15 to about 25 g/m² and an average maximal pore length between 50 $\mu$m and 500 $\mu$m, preferably between 100 and 400 $\mu$m.

The average maximal pore length is measured according to the following protocol: three sample areas of 20 cm × 20 cm are randomly selected and cut out of the nonwoven layer. Each sample area shall not be selected closer than 2 cm to an edge (cutting or production edge) of the nonwoven layer. Each sample area is put on a black support (e.g. on a black paper) and visually inspected for the hole defects. The largest hole defect is selected and marked (e.g. by drawing a circle of about 2.5 cm diameter around the defect), wherein the circle defines the borders of the selected area. The selected area is analyzed with a light microscope with background light and the largest diameter of the free pore areas is visually determined and measured. The highest measurement of all three sample areas determines the maximal pore length and the average of the three measurements corresponds to the average maximal pore length.

It has been found that such a nonwoven layer is highly effective for preventing fiber lint when used as a tampon overwrap. Indeed, measurements that were made by the applicant showed a surprisingly good performance of the nonwoven layers when used as a tampon overwrap, wherein the fiber lint was comparable to market samples that use a tampon overwrap comprising synthetic fiber material, such as polyethylene, polypropylene or polyester.

The properties of the novel nonwoven layers disclosed herein can also be used for other applications, not only in the field of hygienic uses, and it is to be noted that the scope of this disclosure is not restricted to tampon overwrap materials or the use for hygienic products.

Generally, "normal" hole defects (i.e. hole defects that cannot be avoided and appear normally in the specific production process) are located in a random manner spread over the whole area of the nonwoven product. Therefore a measurement of only three randomly selected sample areas gives a sufficiently good characterization of the overall quality of the nonwoven layer.

The term "substantially continuous regenerated cellulosic fibers", as it is used herein, refers to fibers that are produced in a continuous spinning process, wherein the fibers are not cut into staple fibers. Preferably the production of the substantially continuous regenerated cellulosic fibers comprises a solution blown process.

Due to the use of substantially continuous regenerated cellulosic fibers, the nonwoven layer non only has the capability of protecting an absorbent core from fiber lint, but also has a low fiber lint itself.

According to one embodiment, the nonwoven layer has an average maximal free pore area between about 2000 $\mu$m² and about 100.000 $\mu$m², preferably between about 4.000 $\mu$m² and about 60.000$\mu$m².

The average maximal free pore area is measured essentially according to the same protocol as described above in connection with the measurement of the maximal pore length, only that instead of the largest diameter, the area of the free pore area is measured by free-hand polygonal selection. The average maximal free pore area is determined as the average of the three measurements taken.

The average maximal free pore area is closely linked to the average maximal pore length, but also takes into account the form of the pore area. Obviously, a "flat" or elliptical pore shape has a lower pore area than a round pore shape of the same maximal diameter.

According to a further embodiment, the fibers in the nonwoven layer are finish free and essentially free of odorous substances.

For hygiene articles the purity of the materials that are in direct contact with the body is very important to the users. The nonwoven material according to the present disclosure can be produced essentially free of contaminates, such as odorous substances. Also, no finishing agent need to be applied to obtain a completely finishing free material.

"Finish free", as the term is used herein, denotes fibers that have not been subjected to a finishing step and therefore are free of finishing agents.

The term "odorous substances", as it is used herein, denotes substances that cause a scent in the end-product that can be perceived by end users.

The term "essentially free of odorous substances" as it is used herein, denotes that these substances are either not present in the nonwoven material or maximally present in practically negligible amounts, i.e. that they cannot be perceived in the end product by a majority of the end users

In one embodiment, the nonwoven layer can be provided with at least one finishing agent and/or with at least one active substance.

The finishing agent can, for example, be selected from a list comprising a friction reducing finishing or a softening finishing. The active substance can be selected from a list comprising fragrances, control agents, anti-bacterial agents, hydrophobizing agents for controlled fluid management or the like.

According to one embodiment, the nonwoven layer can be essentially free of copper and/or nickel.

This can be realized by the use of operating fluids (in particular lyocell spinning solution, coagulation fluid, washing liquor, gas flow, etc.) during the manufacturing process that are substantially free of heavy metal sources such as copper salt. As a result of this design of the manufacturing process, the cellulosic fibers according to the present disclosure may be of high quality and may substantially consist of pure microfibrillar cellulose. The absence of any mentionable heavy metal impurities in the manufacturing process prevents highly undesired decomposition of involved media (in particular of the lyocell spinning solution) and therefore allows to obtain highly reproducible and highly pure cellulose fibers that are biodegradable and skin-friendly.

The term "essentially free of copper and/or nickel", as it is used herein, denotes that these substances are maximally present in practically negligible amounts. For example, a copper contents of less than 5 ppm and/or a nickel contents of less than 3 ppm is practically negligible in most cases and therefore can be considered as essentially free of copper and/or nickel, respectively.

In another embodiment, the nonwoven layer can have a basis weight of between 10 and 35 $g/m^2$, preferably between 10 and 20 $g/m^2$.

The inventors found, that the nonwoven materials according to the present disclosure show very good lint retention properties at surprisingly low basis weight, which is preferred for most applications.

In the nonwoven layer according to the present disclosure, at least a part of the nonwoven layer can be directly manufactured from lyocell spinning solution.

Such a nonwoven layer forms a network of substantially endless regenerated cellulosic fibers. The nonwoven layer can easily, yet reliably be produced and can be reproducibly provided with a very homogenous pore size distribution. The nonwoven layer may contain essentially only cellulose, thus, showing good biodegradability. The cellulosic nonwoven layer, directly manufactured from lyocell spinning solution, can be manufactured according to a novel variant of a solution blown process (also called spunlaid nonwoven process), e.g. as described in WO 2018/184038 and WO 2018/184932. In jurisdictions where this is possible, the documents WO 2018/184038 and WO 2018/184932 are included herein by reference. In this process, a (lyocell) cellulose spinning mass is extruded simultaneously through a spinneret containing closely-spaced meltblown jet nozzles. The extruded spinning solution is then attenuated using high velocity air streams and the nonwoven web/layer is formed on a moving surface. Finally the nonwoven web is washed and dried. By carefully adjusting the spinning parameters (cellulose concentration of the spinning mass, spinneret extrusion rate, attenuating air stream velocity, etc.), the strength- and liquid-transport-properties of the formed nonwoven can be tailored to the needs. Such a nonwoven layer formed by the above mentioned process may thus provide good lint retention properties, enhanced dimensional stability (wet and dry), biodegradability and sustainability; all without the need of adding binders or chemicals.

According to one embodiment, the fibers of the carrier layer can have diameters ranging from 1 $\mu$m to 5000 $\mu$m, preferably from 8 $\mu$m to 500 $\mu$m.

This broad scope includes cellulosic materials that are made according to a meltblown process. In this case the larger diameters in particular belong to merging points. Most of the suitable meltblown materials according to the present disclosure may show fiber diameters of up to 500 $\mu$m only, however materials with the larger diameters may be suitable, as well. If the nonwoven cellulosic material is not manufactured by a meltblown process, the diameter range is much narrower, more comparable to that of a nonwovens consisting of staple fibers. In such case the fiber diameter may range from 1 $\mu$m to 100 $\mu$m, preferably from 7 $\mu$m to 50 $\mu$m.

According to another embodiment, the cellulosic fibers in the nonwoven layer can be multibonded by merging, hydrogen bonding and/or physically intermingling.

Thus, the dimensional stability of the nonwoven layer may be improved, which also improves the machinability of the nonwoven layer.

In another embodiment, at least one surface of the nonwoven layer can have heat sealable properties.

This facilitates more economic production processes, e.g. when the nonwoven layer is used as a tampon overwrap. The heat-sealability can be provided by several techniques which may be known per se in the art, such as admixing heat sealable fibers to the nonwoven layer or adding a thin layer of a heat-sealable nonwoven or film material to at least one surface of the nonwoven layer, etc. As a sealable material biodegradable materials can be preferred, such as Polyhydroxyalkanoates or polylactic acid.

The nonwoven layer according to the present disclosure can comprise a network of substantially continuous regenerated cellulosic fibers as a tampon overwrap.

This allows for a cost efficient application of a tampon overwrap with beneficial properties, e.g. biodegradability. The use of the nonwoven layers according to the present disclosure offers the sustainability benefit combined with functionality as they can be homogeneously produced even at low basis weight.

According to another aspect, the present disclosure relates to a tampon comprising a nonwoven layer according to the present disclosure as a tampon component.

Apart from the beneficial fiber lint retaining properties, the nonwoven layer used as a tampon overwrap takes up and distributes liquid fast. Also, the nonwoven layers according to the present disclosure provide for a soft and silky surface, to allow an easy use and removal. Additionally, the nonwoven layers according to the present disclosure can also be beneficially used for other components of the tampon.

In a further aspect, the present disclosure relates to a method for production of a tampon, comprising at least one step of at least partly enclosing an absorbent core with a tampon overwrap comprising a nonwoven layer according to the present disclosure.

**Brief Description of the Drawings**

Hereinafter, exemplary embodiments of the invention are described with reference to the drawings, wherein

Fig. 1      shows a microscopic picture of a part of Innovative Sample A according to the present disclosure,

Fig. 2      shows a microscopic picture of a part of Innovative Sample B according to the present disclosure

Fig. 3      shows a microscopic picture of a part of Comparison Sample C,

Fig. 4      is photographic depiction of a sample area of a thin nonwoven layer according to the state of the art,

Fig. 5      shows an enlarged section of the picture shown in Fig. 4 comprising a selected area marked by a circle,

Fig. 6      is photographic depiction of a sample area of a thin nonwoven layer according to the present disclosure,

Fig. 7      shows an enlarged section of the picture shown in Fig. 6 comprising a selected area marked by a circle,

Fig. 8      is a schematic representation illustrating the steps for measuring the pore area of a pore or hole defect, respectively,

Fig. 9      is a schematic representation illustrating the steps for measuring the pore length of a pore or hole defect, respectively

Fig. 10      shows a tampon comprising a core and a tampon overwrap before being pressed, the overwrap comprising a nonwoven layer according to the present disclosure.

**Examples and detailed description of the drawings**

**Example 1 - Pore sizes**

Two samples according to the present disclosure (Innovative Sample A and Innovative Sample B) and one Comparative Sample (Sample C) according to the specifications disclosed below in Table 1 were analyzed according to the protocol described below.

Innovative Sample A and Innovative Sample B were both produced in an industrial scale prototype facility at the premises of Lenzing AG, Lenzing, Austria, according to a solution blown process as disclosed, e.g., in WO 2007/124521 A1. About 10 meters of each Sample were produced, each having a width of about 0.45 m. The production parameters were chosen to optimize the uniformity of the pore size distribution. Two grades of filament fineness distributions were generated by varying the stretching air. For Sample B, 1.27 times the stretching air of Sample A was used. The resulting higher fraction of fine filaments in Sample B led to a lower average pore length and to a lower average pore area in Sample B. The filaments of Sample A and Sample B were bound by merging, hydrogen bonding and physical intermingling to form the nonwoven web.

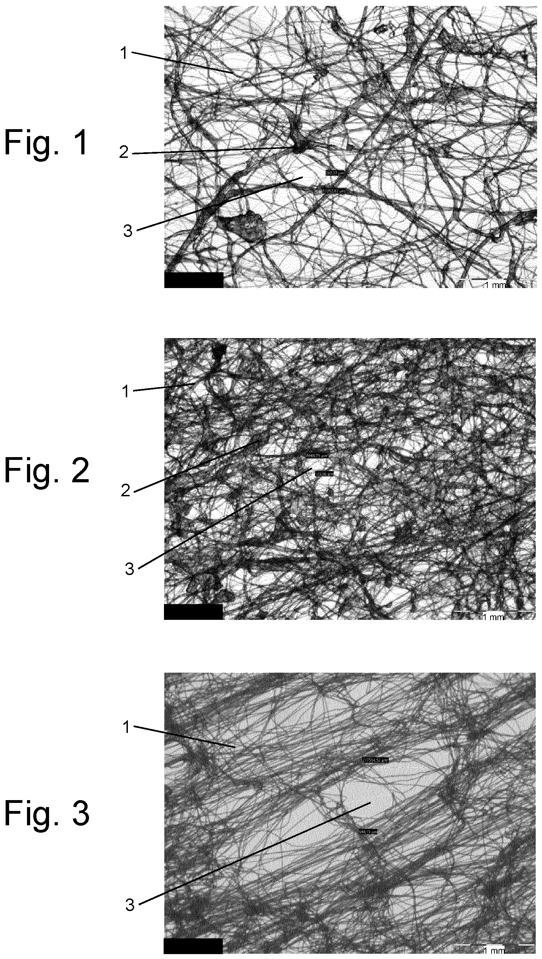

Fig. 1 shows a microscopic picture of a part of a sample area of a nonwoven layer taken from Innovative Sample A. The nonwoven layer depicted in Fig. 1 has a length of about 4.7 mm and a width of about 3.6 mm. The pattern of the fibers 1 and the merging points 2 define free pore areas 3 which can be measured and characterized according to the methods disclosed herein.

Fig. 2 shows a microscopic picture of a part of a sample area of a nonwoven layer taken from Innovative Sample B. The nonwoven layer depicted in Fig. 2 has a length of about 4.7 mm and a width of about 3.6 mm.

It can be seen by a comparison of Fig. 1 and 2 that Innovative Sample B has a denser structure and generally smaller pore areas than Innovative Sample A. Nonetheless, both samples have a very uniform distribution of pore sizes.

As the Comparative Sample C, a common cellulosic staple fiber fabric - 1.7 dtex, 38 mm, dull - was selected. The cellulosic staple fiber fabric according to Comparative Sample C was produced by a conventional carding and hydroentangling process.

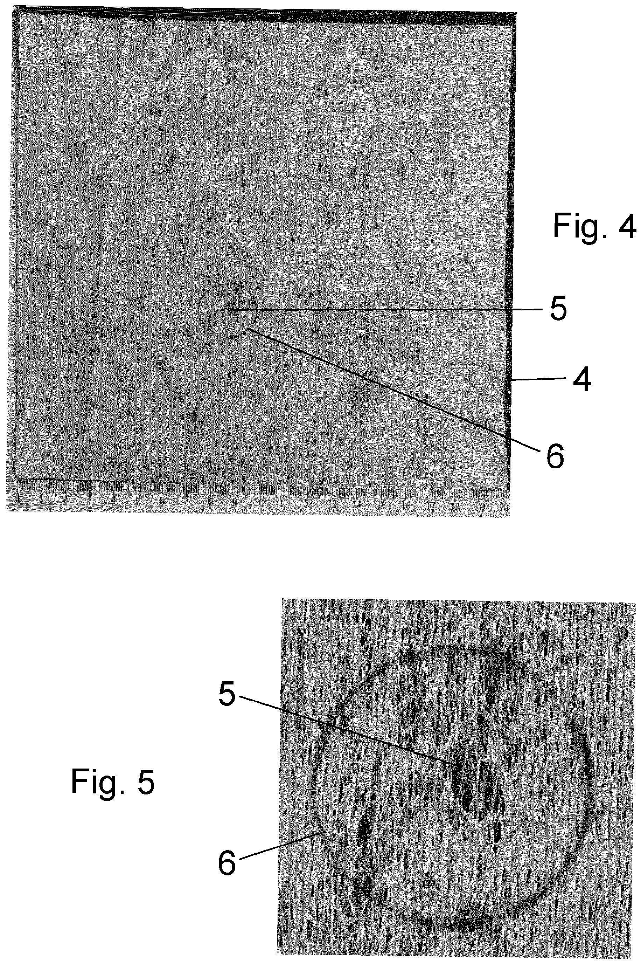

Fig. 3 shows a microscopic picture of a part of a sample area of a nonwoven layer taken from Comparative Sample C. The nonwoven layer depicted in Fig. 3 has a length of about 4.7 mm and a width of about 3.6 mm. As can be seen from the depiction in Fig. 3, the conventional nonwoven fabric shows rather large pore size differences, even on the microscopic scale. As will be shown in Fig. 4 and 5, the pore sized of these fabrics differ even more on a macroscopic scale (i.e. visible to the bare eye).

All samples were conditioned at 23°C (±2°C) and 50% (±5%) relative humidity for 24 hours.

The mass per unit area (basis weight) was determined according to Nonwoven Standard Procedure NWSP 130.1.R0 (15).

The thickness was determined according to NWSP 120.6.R0 (15) - Option A.

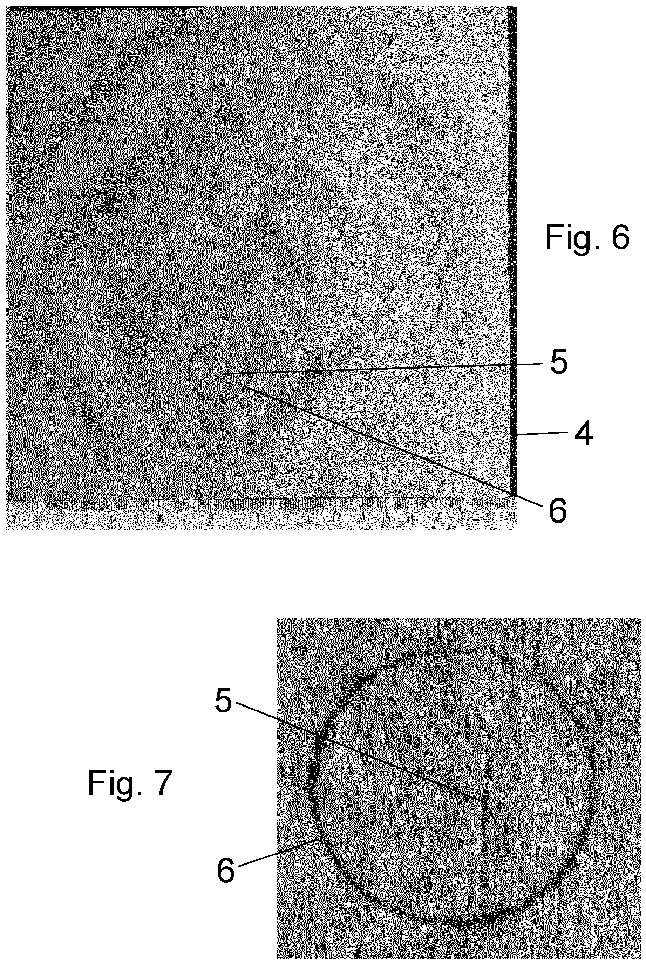

Of each fabric type three 20x20 cm sample areas were randomly selected and cut out. The fabrics were placed flat on a black A4 size paper and visually checked for the largest hole defect. Fig. 4 shows such a sample area 4 of a cellulosic staple fiber nonwoven fabric according to Comparative Sample C and Fig. 6 shows a sample area 4 of a nonwoven layer according to Innovative Sample B. The visually determined area with the largest hole defect 5 was marked with a circle 6 in each 20 x 20 cm sample area (see Fig. 5 for an enlarged depiction of the selected area marked by the circle 6 of Comparative Sample C and Fig. 7 for an enlarged depiction of the selected area marked by the circle 6 of Innovative Sample B). In the pictures shown in Fig. 4 to 7 the circle 6 has a diameter of about 2.5 cm. The hole defects (i.e. the largest free pore area 3 in the selected area marked by the circle 6) were further investigated and measured under light microscope (Stereo Microscope Zeiss Discovery.V12) using the following settings/parameters:

Light setting: Transmitted light: Zeiss CL 9000 LED

Camera: Olympus XC 50 with adapter 60N-C 2/3 0.63x 426113

Software: Olympus Stream Motion 2.4.2 (Build 20105)

Software setting:

- Automatic contrast

- Automatic exposure time (light)

- Measurement of maximum free pore area in the fabric with function "Freihandpolygon" ("freehand polygon")

- Measurement of maximal length of free area (maximal pore length) with function "beliebige Länge" ("optional length")

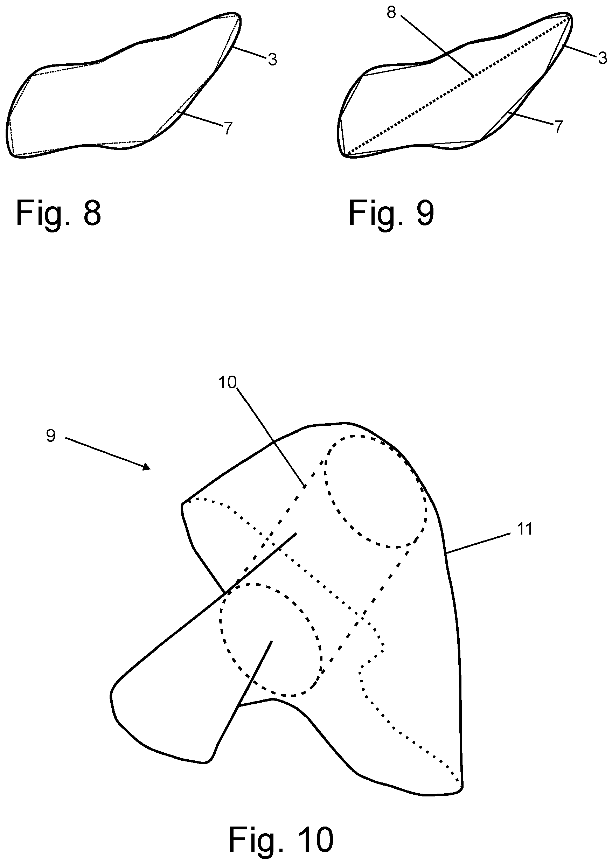

Fig. 8 shows a schematic depiction of the measurement function "freihandpoygon": A closed polygon 7 (represented by a dottet line) is inscribed by mouse pointer selection into a free pore area 3 (which is represented by a continuous line in Fig. 8). The software calculates the area of the selected polygon, which is saved as the measurement value for the free pore area 3 of the pore.

The software further calculates the longest diameter 8 between two points of the perimeter of the closed polygon 7, which is saved as the measurement value for the pore length of the pore. The pore length (i.e. the longest diameter 8) is exemplarily depicted by the dotted line in Fig. 9.

The measurement values of all three sample areas per sample type for maximal free pore area and maximal pore length were averaged to determine the average maximal pore length and the average maximal free pore area, respectively. Further, the maximum value of any of the three sample areas determined the maximal pore length of the sample type.

The parameters and the measurement results of the samples are shown in Table 1.

Table 1 - Pore size measurement results

| Sample | Basis weight [g/m$^2$] | Thickness [mm] | Pore length [μm] | | Pore area [μm$^2$] | |
|---|---|---|---|---|---|---|
| | | | Average | Maximum | Average | Maximum |
| Innovative Sample A | 17 | 0.25 | 326.4 | 400.2 | 57,976.4 | 62,992.2 |
| Innovative Sample B | 21 | 0.19 | 165.2 | 224.7 | 15,405.2 | 26,472.8 |
| Comparative Sample C | 25 | 0.33 | 699.6 | 780.7 | 175,679.7 | 217,554.5 |

The pore size and area measurements show that the nonwoven fabric according to the present disclosure (represented by Innovative Samples A and B) show a considerably reduced maximal pore length and maximal pore area as compared to a common thin cellulosic staple fiber nonwoven fabric (as represented by Comparative Sample C).

**Example 2 - Tampon generation and determination of fiber loss**

For assessing the fiber loss of a tampon comprising a nonwoven layer according to the present disclosure as a tampon overwrap, test-tampons were produced and tested according to the protocols described below. Fig. 10 schematically shows the structure of such a tampon 9, which comprises an absorbent core 10 and a tampon overwrap 11, immediately before compounding them by pressing into a tampon form.

As a general rule, all cellulosic test materials were conditioned at 23°C (±2°C) and 50% (±5%) relative humidity for 24 hours prior to testing.

For the tampon generation the following cellulosic materials were prepared:
Staple fiber RM - Viscostar 3.3 dtex, 30 mm cut length, bright as reference material for tampon without overwrap and as absorbent core material for the tampons with a tampon overwrap.

Sample Tampons A, Sample Tampons B and Comparative Sample Tampons D were produced.

Sample Tampons A comprised the absorbent core and a tampon overwrap made from a 100 % cellulosic nonwoven material of essentially endless filaments according to the nonwoven layer of Innovative Sample A in Example 1.

Sample Tampons B comprised the absorbent core and a tampon overwrap made from a 100 % cellulosic nonwoven material of essentially endless filaments according to the nonwoven layer of Innovative Sample B in Example 1

Comparative Sample Tampons D comprised an absorbent core without a tampon overwrap.

For the overwrap fabrics the Mass per Unit Area (basis weight) was determined according to NWSP 130.1.R0 (15). Of each fabric type two 130 cm diameter circles were cut and weighed to generate the overwrap for the tampons.

For generation of the reference tampon material and the tampon filling material to be combined with the overwraps, six card slivers (rings) were generated with a laboratory rotor-ring card (model USTER MDTA3). For each card sliver ring 2.6 g of staple fibers were used. The generated card sliver rings were each opened (approximately 90 cm length) and folded to 3 layers of approximately 30 cm length.

The generated three layer card slivers were each prepressed on a card sliver press with 3 Nm roll pressure. Then, the exact weight of 2.5 g for the reference tampon material was adjusted by removing access fibers from the sides of the prepressed card slivers. This was done twofold to generate a double determination of the reference tampon material. For the composites with overwrap the access amount of staple fiber was removed from the prepressed card slivers to generate a 2.5 g tampon comprising the filler fiber weight (i.e. the weight of the absorbent core) and the respective weight of the tampon overwrap. For each material this step also was done twofold.

All the prepressed card slivers were wound with a winding device under a weight of 150g. For the reference tampons, the wound card slivers were pressed with a tampon press (type Ruggli Fertigpresse - model "Occasion Linearpresse", eightfold combination jaw - regular, orifice 29-4-044 ø 13/17/11) for 2 minutes.

For the composites with tampon overwrap the wound card slivers were combined with the circular overwrap by covering the top of the wound card sliver with the center point of the circular tampon overwrap and then folding the overwrap over the wound card sliver sides (this corresponds to the depiction in Fig. 10).

The composite products (i.e. the absorbent core 10 and the tampon overwrap 11) were pressed with the same tampon press (type Ruggli Fertigpresse) for 2 minutes each.

All the generated tampon products were subsequently immediately analyzed for fiber loss during a wet shaking process according to the protocol described below.

The generated tampon plugs (composite plugs and reference plugs) were placed each in a 600 ml volume beaker glass with 500 ml of deionized water and were shaken for 10 min with 100 rpm on shaker SM-30 (Edmund Bühler Gmbh).

Folded filters (595 1/2 LOT Nr. A10434264 Whatman), each previously dried for 12 h in a drying chamber (type Heraeus Kelvitron T) at 105 °C and weighed afterwards on an analytical balance (type Sartorius analytic) were used for filtering the water with the fiber residues after the shaking process.

The folded filters with the fiber residues were then again dried for 12 h in the drying chamber (type Heraeus Kelvitron T) at 105 °C and weighed afterwards on an analytical balance (type Sartorius analytic).

The amount of fiber loss was calculated according to the following formula:

$$\text{Fiber loss [g]} = \text{dry weight of folded filter with dry fiber residue} - \text{dry weight of folded filter}$$

The fiber loss values of the double determinations per composite with overwrap and per reference without overwrap were averaged. The results are shown in Table 2 below.

Table 2 - Tampon Fiber Loss

|  | fiber loss [g] determination one | fiber loss [g] determination two | Average of fiber loss [g] |
|---|---|---|---|
| Sample Tampon A | 0.0099 | 0.0146 | 0.0123 |
| Sample Tampon B | 0.0179 | 0.0080 | 0.0130 |
| Comparative Sample Tampon D | 0.0331 | 0.0592 | 0.0462 |

Within this experiment the Sample Tampons A and B, each comprising a tampon overwrap of 100 % cellulosic nonwoven material of essentially endless filaments according to the nonwoven layer of the present disclosure showed a significant reduction of fiber loss during the wet shaking test compared to Comparative Sample Tampon D.

Reference numbers:

fibers 1
merging points 2
free pore area 3
sample area 4
largest hole defect 5
circle 6
closed polygon 7
longest diameter 8
tampon 9
absorbent core 10
tampon overwrap 11

**Claims**

1.   Nonwoven layer comprising a network of substantially continuous regenerated cellulosic fibers, **characterized in that** the nonwoven layer has a basis weight from about 10 to about 30 $g/m^2$, preferably from about 15 to about 25 $g/m^2$ and an average maximal pore length between 50 $\mu$m and 500 $\mu$m, preferably between 100 and 400 $\mu$m.

2.   Nonwoven layer according to Claim 1, wherein the nonwoven layer has an average maximal free pore area between about 2000 $\mu m^2$ and about 100.000$\mu m^2$, preferably between about 4.000 $\mu m^2$ and about 60.000$\mu m^2$.

3.   Nonwoven layer according to Claim 1 or 2, wherein the fibers in the nonwoven layer are finish free and essentially free of odorous substances.

4. Nonwoven layer according to Claim 1 or 2, wherein the nonwoven layer is provided with at least one finishing agent and/or with at least one active substance.

5. Nonwoven layer according to any of the Claims 1 to 4, wherein the nonwoven layer is essentially free of copper and/or nickel.

6. Nonwoven layer according to any of the Claims 1 to 5, wherein the nonwoven layer has a basis weight of between 10 and 35 g/m$^2$, preferably between 10 and 20 g/m$^2$.

7. Nonwoven layer according to any of the Claims 1 to 6, wherein at least a part of the nonwoven layer is directly manufactured from lyocell spinning solution.

8. Nonwoven layer according to Claim 7, wherein the fibers of the carrier layer have diameters ranging from 1 $\mu$m to 5000 $\mu$m, preferably from 8 $\mu$m to 500 $\mu$m.

9. Nonwoven layer according to any of the Claims 1 to 8, wherein the cellulosic fibers in the nonwoven layer are multibonded by merging, hydrogen bonding and/or physically intermingling.

10. Nonwoven layer according to any of the Claims 1 to 9, wherein at least one surface of the nonwoven layer has heat sealable properties.

11. Use of a nonwoven layer, preferably according to any of the Claims 1 to 10, comprising a network of substantially continuous regenerated cellulosic fibers as a tampon overwrap.

12. Tampon comprising a nonwoven layer according to any of the Claims 1 to 10 as a tampon component.

13. Method for production of a tampon, comprising at least one step of at least partly enclosing an absorbent core with a tampon overwrap comprising a nonwoven layer according to any of the Claims 1 to 10.

Fig. 1

Fig. 2

Fig. 3

Fig. 4

— 5

— 4

— 6

Fig. 5

5

6

Fig. 6

Fig. 7

Fig. 8

Fig. 9

Fig. 10

Europäisches
Patentamt

European
Patent Office

Office européen
des brevets

## EUROPEAN SEARCH REPORT

Application Number

EP 22 15 2553

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2018/184932 A1 (CHEMIEFASER LENZING AG [AT]) 11 October 2018 (2018-10-11) | 1-10 | INV. A61F13/20 |
| A | * page 22, line 12 - line 27 *<br>* page 31, line 29 - page 32, line 5 *<br>* page 32, line 30 - page 33, line 29 *<br>* page 34, line 25 - line 27 *<br>* page 35, line 2 - line 7 * | 11-13 | D04H1/4258<br>D04H3/013 |
| | ----- | | |
| X | US 2020/102676 A1 (CARLYLE TOM [US] ET AL) 2 April 2020 (2020-04-02) | 1-10 | |
| A | * paragraph [0096] - paragraph [0105]; claims 1, 2, 5; figure 1 * | 11-13 | |
| | ----- | | |
| X | WO 2018/184049 A1 (CHEMIEFASER LENZING AG [AT]) 11 October 2018 (2018-10-11) | 1-10,12 | |
| A | * page 4, line 4 - line 7; claims 7, 13, 15 * | 11,13 | |
| | ----- | | |
| X,D | EP 1 093 536 B1 (TENCEL LTD [GB]) 1 October 2003 (2003-10-01) | 1-10 | |
| A | * paragraph [0023] - paragraph [0034]; figure 1 * | 11-13 | TECHNICAL FIELDS SEARCHED (IPC) |
| | ----- | | A61F |
| X,D | WO 2007/124521 A1 (CHEMIEFASER LENZING AG [AT]; WHITE PAT [GB] ET AL.) 8 November 2007 (2007-11-08) | 12 | D04H |
| A | * page 7 - page 9 * | 1-11,13 | |
| | ----- | | |
| X,D | EP 2 212 456 B1 (CHEMIEFASER LENZING AG [AT]) 22 July 2015 (2015-07-22) | 12 | |
| A | * paragraph [0021] *<br>* paragraph [0025] *<br>* paragraph [0064] *<br>* paragraph [0071] * | 1-11,13 | |
| | ----- | | |
| | -/-- | | |

The present search report has been drawn up for all claims

| Place of search | Date of completion of the search | Examiner |
|---|---|---|
| Munich | 30 June 2022 | Demay, Stéphane |

EPO FORM 1503 03.82 (P04C01)

Europäisches
Patentamt

European
Patent Office

Office européen
des brevets

**EUROPEAN SEARCH REPORT**

Application Number

EP 22 15 2553

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2009/312731 A1 (STEINDL THOMAS [AT] ET AL) 17 December 2009 (2009-12-17)<br>* paragraph [0071] - paragraph [0074]; claim 23 *<br>----- | 11,12<br>1-10,13 | |
| A,D | WO 2018/184038 A1 (CHEMIEFASER LENZING AG [AT]) 11 October 2018 (2018-10-11)<br>* the whole document *<br>----- | 1-13 | |
| | | | **TECHNICAL FIELDS SEARCHED (IPC)** |

The present search report has been drawn up for all claims

| Place of search | Date of completion of the search | Examiner |
|---|---|---|
| Munich | 30 June 2022 | Demay, Stéphane |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 15 2553

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2022

| Patent document cited in search report | | | Publication date | Patent family member(s) | | | Publication date |
|---|---|---|---|---|---|---|---|
| WO 2018184932 | A1 | | 11-10-2018 | BR | 112019020741 | A2 | 28-04-2020 |
| | | | | CN | 110651077 | A | 03-01-2020 |
| | | | | EP | 3385433 | A1 | 10-10-2018 |
| | | | | EP | 3607129 | A1 | 12-02-2020 |
| | | | | JP | 7005740 | B2 | 24-01-2022 |
| | | | | JP | 2020515737 | A | 28-05-2020 |
| | | | | KR | 20190130653 | A | 22-11-2019 |
| | | | | TW | 201900966 | A | 01-01-2019 |
| | | | | US | 2018282921 | A1 | 04-10-2018 |
| | | | | WO | 2018184932 | A1 | 11-10-2018 |
| US 2020102676 | A1 | | 02-04-2020 | BR | 112019020736 | A2 | 12-05-2020 |
| | | | | CN | 110603356 | A | 20-12-2019 |
| | | | | EP | 3385426 | A1 | 10-10-2018 |
| | | | | EP | 3607128 | A1 | 12-02-2020 |
| | | | | JP | 7019929 | B2 | 16-02-2022 |
| | | | | JP | 2020515736 | A | 28-05-2020 |
| | | | | KR | 20190130036 | A | 20-11-2019 |
| | | | | TW | 201900968 | A | 01-01-2019 |
| | | | | US | 2020102676 | A1 | 02-04-2020 |
| | | | | WO | 2018184930 | A1 | 11-10-2018 |
| WO 2018184049 | A1 | | 11-10-2018 | NONE | | | |
| EP 1093536 | B1 | | 01-10-2003 | AT | 251239 | T | 15-10-2003 |
| | | | | AU | 4158299 | A | 30-12-1999 |
| | | | | BR | 9910862 | A | 06-03-2001 |
| | | | | CA | 2333882 | A1 | 16-12-1999 |
| | | | | CN | 1304464 | A | 18-07-2001 |
| | | | | DE | 69911776 | T2 | 19-08-2004 |
| | | | | EP | 1093536 | A1 | 25-04-2001 |
| | | | | ES | 2209444 | T3 | 16-06-2004 |
| | | | | GB | 2337957 | A | 08-12-1999 |
| | | | | ID | 27038 | A | 22-02-2001 |
| | | | | JP | 4373008 | B2 | 25-11-2009 |
| | | | | JP | 2002517630 | A | 18-06-2002 |
| | | | | KR | 20010071345 | A | 28-07-2001 |
| | | | | TR | 200003602 | T2 | 20-04-2001 |
| | | | | TW | 419543 | B | 21-01-2001 |
| | | | | WO | 9964649 | A1 | 16-12-1999 |
| WO 2007124521 | A1 | | 08-11-2007 | AT | 503625 | A1 | 15-11-2007 |
| | | | | CN | 101432476 | A | 13-05-2009 |
| | | | | CN | 103173938 | A | 26-06-2013 |
| | | | | EP | 2013390 | A1 | 14-01-2009 |
| | | | | EP | 2957667 | A1 | 23-12-2015 |

EPO FORM P0459

For more details about this annex : see Official Journal of the European Patent Office, No. 12/82

**page 1 of 2**

## ANNEX TO THE EUROPEAN SEARCH REPORT
## ON EUROPEAN PATENT APPLICATION NO.

EP 22 15 2553

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2022

| Patent document cited in search report | | Publication date | Patent family member(s) | | Publication date |
|---|---|---|---|---|---|
| | | | ES | 2553182 T3 | 04-12-2015 |
| | | | JP | 5097771 B2 | 12-12-2012 |
| | | | JP | 2009535521 A | 01-10-2009 |
| | | | KR | 20080111521 A | 23-12-2008 |
| | | | PL | 2013390 T3 | 31-12-2015 |
| | | | US | 2009186189 A1 | 23-07-2009 |
| | | | WO | 2007124521 A1 | 08-11-2007 |
| EP 2212456 | B1 | 22-07-2015 | AT | 505621 A4 | 15-03-2009 |
| | | | CN | 101896656 A | 24-11-2010 |
| | | | EP | 2212456 A1 | 04-08-2010 |
| | | | ES | 2550279 T3 | 05-11-2015 |
| | | | JP | 5425088 B2 | 26-02-2014 |
| | | | JP | 2011503368 A | 27-01-2011 |
| | | | KR | 20100080606 A | 09-07-2010 |
| | | | PL | 2212456 T3 | 30-11-2015 |
| | | | US | 2011124258 A1 | 26-05-2011 |
| | | | WO | 2009059342 A1 | 14-05-2009 |
| US 2009312731 | A1 | 17-12-2009 | EP | 2013385 A1 | 14-01-2009 |
| | | | US | 2009312731 A1 | 17-12-2009 |
| | | | WO | 2007124522 A1 | 08-11-2007 |
| WO 2018184038 | A1 | 11-10-2018 | BR | 112019020010 A2 | 28-04-2020 |
| | | | CN | 110506139 A | 26-11-2019 |
| | | | EP | 3607123 A1 | 12-02-2020 |
| | | | JP | 2020521061 A | 16-07-2020 |
| | | | KR | 20190127975 A | 13-11-2019 |
| | | | TW | 201843368 A | 16-12-2018 |
| | | | US | 2021101362 A1 | 08-04-2021 |
| | | | WO | 2018184038 A1 | 11-10-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1093536 B1
- EP 2013390 B1
- EP 2212456 B1
- WO 2007124521 A1
- WO 2018071928 A1
- WO 2018184941 A1
- WO 2020016296 A1
- EP 3730111 A1
- WO 2018184038 A
- WO 2018184932 A